# EUROPEAN PATENT APPLICATION

(11) **EP 1 917 910 A2**
(43) Date of publication of application: **07.05.2008**
(21) Application number: 07015873.8
(22) Date of filing: 13.08.2007
(51) Int. Cl.: A61B 5/151

(54) **Safety lancet**

(30) Priority: 06.11.2006 CN 200620157140 U
(71) Applicant: Apex Biotechnology Corporation, Hsinchu City 300 (TW)
(72) Inventor: Yang, Moon Wen, Hsinchu City 300 (TW); Hsu, Ming-Chang, Hsinchu City 300 (TW); Lin, Yueh-Hui, Hsinchu City 300 (TW); Wang, Jui-Ping, Hsinchu City 300 (TW); Shen, Thomas Y., Hsinchu City 300 (TW)
(74) Representative: Flaccus, Rolf-Dieter

(57) **Abstract**

A safety lancet (100) includes a housing (120), an actuating unit (140), a needle unit (160), a blocking unit (180) and an elastic unit (190). The housing has a side hole (122) and a front opening (124). The actuating unit is disposed on an outer surface of the housing. The needle unit, including a needle (162), a flex pin (164) and a blocking pin (166), is disposed inside the housing. The needle corresponds to the front opening and the flex pin corresponds to the side hole. One end of the flex pin extends into the side hole to position the needle unit at a first position. The blocking unit is disposed inside the housing and corresponds to the blocking pin to allow the needle unit to forwardly pass through the blocking unit and prevent the needle unit from backwardly passing through the blocking unit.

## Description

### Cross Reference to Related Applications

This application claims priority of China Patent Application No. 200620157140.0 entitled "SAFETY SYRINGE", filed on November 6, 2006.

### Field of the Invention

The present invention relates to a safety lancet, and more particularly to a safety lancet with a single puncture mode to prevent from being reused.

### Background of the Invention

In current medical industry, all medical personnel use or are exposed to puncture devices or other invasive devices almost everyday, such as blood lancet, injection syringe, etc. Therefore, the safety issues for relevant personnel should be carefully addressed in order to prevent users from accidentally touching used needles as well as intentionally reusing these devices.

Some safety lancet devices have be disclosed, such as a safety lancet device with two puncture modes to accomplish the safety mechanism disclosed in US Patent No. 6,945,982. Such lancet device includes a sleeve body having a front elongate portion and a rear elongate portion. In the first mode, the first elongate portion is forwardly pushed. In the second mode, the second elongate portion is retained at an extended position. Such a complicated mechanism causes a relative high manufacturing cost.

In addition, another conventional device includes a movable sleeve positioned by a surrounded mechanism, such as disclosures in US Patent No. 4,813,940 and No. 5,104,384. A needle, covered by a protective sleeve, need to be exposed before using said device. When the needle is inserted into a patient, the sleeve immediately contacts the patient and a retracting force provided by a spring forces it back to a contracted position. After the lancet is used, the needle is positioned and locked at an extended position by a locking mechanism. However, this type of lancet has a problem that the lock mechanism on the track is fragile and easily broken, resulting in a risk of exposing the needle.

Therefore, it is advantageous to provide a novel safety lancet device to overcome the inconveniences and limitations of prior arts to provide a one-time usable, lighter, disposable, and safer lancet device.

### Summary of the Invention

In order to overcome the problems of prior arts, the present invention provide a safety lancet device with a simple puncture mechanism to prevent the lancet from being reused either intentionally or accidentally.

One aspect of the present invention is to provide a safety lancet device with the characteristics of simple mechanism, easy assembly, and robusto

Another aspect of the present invention is to provide a safety lancet with a blocking unit to prevent from intentionally reusing the lancet.

The present invention discloses a safety lancet including a housing, an actuating unit, a needle unit, a blocking unit and an elastic unit. The housing has a side hole and a front opening. The actuating unit is disposed on the outer surface of the housing. The needle unit, which includes a needle, a flex pin and a blocking pin, is disposed inside the housing. The needle corresponds to the front opening and the flex pin corresponds to the side hole. One end of the flex pin extends into the side hole to locate the needle unit at a first position. The blocking unit is disposed inside the housing and corresponds to the blocking pin to allow the needle unit to forwardly pass through the blocking unit and prevent the needle unit from backwardly passing through the blocking unit. The elastic unit connects the needle unit and the housing. When an external force is exerted on the actuating unit, the actuating unit pushes the flex pin passing through the side hole and the elastic unit pushes the needle unit passing through the block unit to a second position.

### Brief Description of the Drawings

FIG. 1 is a safety lancet device according to one embodiment of the present invention, illustrating the safety lancet device in a preparation condition; and

FIG. 2 is a safety lancet device according to one embodiment of the present invention, illustrating the safety lancet device in an actuated condition.

### Detailed Description of the Invention

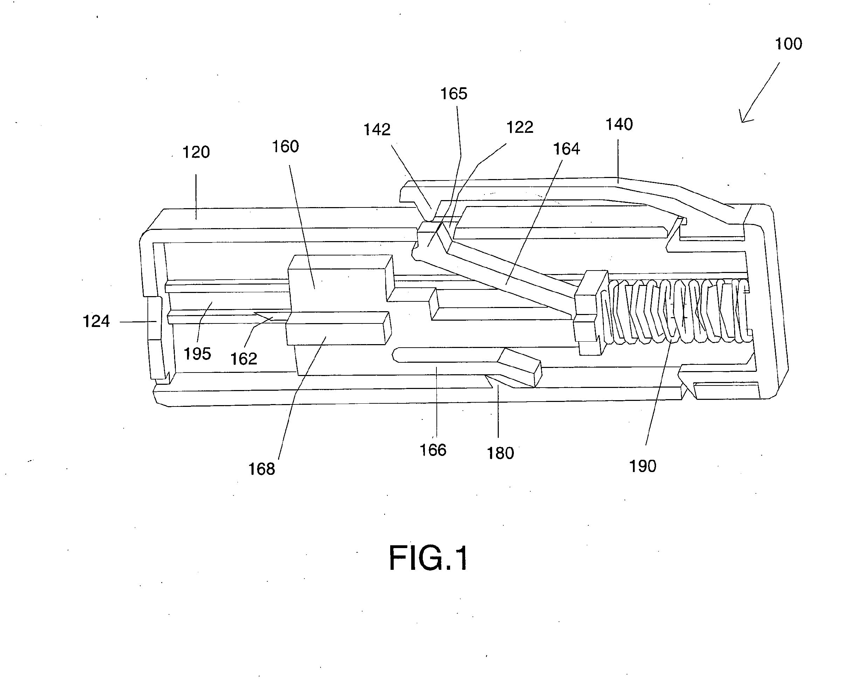

As described above, the present invention discloses a safety lancet device configured to, for example, inject the drug to or sample blood from patients. FIG. 1 is a safety lancet device 100 in a preparation condition according to one embodiment of the present invention. The safety lancet device 100 includes a housing 120, an actuating unit 140, a needle unit 160, a blocking unit 180, and an elastic unit 190. The housing 120 has a side hole 122 and a front opening 124. In this embodiment, the housing 120 is assembled from a first housing and a second housing. For example, FIG. 1 only shows the first housing of the housing 120 for better understanding to the present invention. The second housing (not shown) of the housing 120 has a shape corresponding to the first housing of housing 120. As such, the housing 120 may be easily assembled, thus simplifying the manufacturing process and reducing the manufacturing cost. It is known to those who skilled in the art, however, the housing 120 may be formed integrally in another embodiment of the present invention. The actuating unit 140 is disposed on the outer surface of the housing 120. The needle unit 160, having a needle 162, a flex pin 164 and a blocking pin 166, is disposed inside the housing 120. The needle 162 corresponds to the front opening 124 and the flex pin 164 corresponds to the side hole 122. One end of the flex pin 164 extends into the side hole 122 to locate the needle unit 160 at a first position, as shown in FIG. 1. The blocking unit 180 is disposed inside the housing 120 and corresponds to the blocking pin 166 to allow the needle unit 160 to forwardly pass through the blocking unit 180 and prevent the needle unit 160 from backwardly passing through the blocking unit 180. The elastic unit 190 connects the needle unit 160 and the housing 120. In one embodiment, for example, the elastic unit 190 may be a spring to provide an elastic force to the needle unit 160.

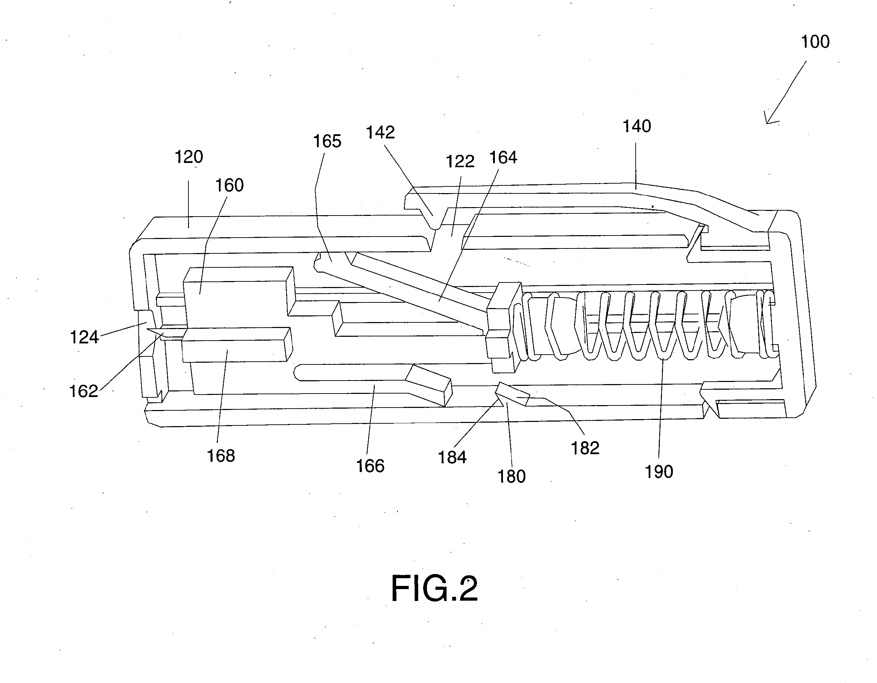

FIG. 2 is the safety lancet device 100 in an actuated condition according to one embodiment of the present invention. When an external force is exerted on the actuating unit 140, the actuating unit 140 pushes the flex pin 164 passing through the side hole 122 and the elastic unit 190 pushes the needle unit 160 passing through the block unit 180 to a second position, as shown in FIG. 2. The blocking unit 180 has a guiding surface 182 and a blocking surface 184. The guiding surface 182 guides the blocking pin 166 to allow the needle unit 160 to forwardly move from the first position (as shown in FIG. 1) to the second position (as shown in FIG. 2). Contrarily, the blocking surface 184 blocks the blocking pin 166 to limit the needle unit 160 backwardly moving from the second position to the first position. Therefore, by means of a simple blocking unit 180, such as the blocking unit 180 protruding from an inner surface of the housing 120 to form a hook shape as shown in the drawings, it can prevent the needle 162 inside the lancet device 100 from accidentally hurting the user or being restored to the preparation condition (i.e. the first position shown in FIG. 1). Although the device is described as a blood lancet in this embodiment, it should be noted that the present invention is not limited to the specific application. That is, the present invention may also be embodied as other puncture device, sampling device, or injection device, such as a syringe.

Further referring to FIG. 1, the present invention may further include a track 195 disposed inside the housing 120 and the needle unit 160 further includes a bulge 168 corresponding to the track 195 to facilitate the needle unit 160 sliding inside the housing 120. One end of the actuating unit 140 connects to the outer surface of the housing 120, and the other end of the actuating unit 140 has a protrusion 142 corresponding to the side hole 122. When an external force is exerted on the actuating unit 140, the protrusion 142 of the actuating unit 140 pushes the flange 165 of the flex pin 164 in the side hole 122, thus the flange 165 is retreated from the side hole 122. Therefore, the needle unit 160 may slide in the housing 120 via the interaction between the bulge 168 and the track 100. When the needle unit 160 moves to the second position, the needle 162 passes through the front opening 124 to be exposed to outside environment of the housing 120.

The above description sets forth various preferred embodiments of the invention only, and is not intended to limit the scope, applicability, or configuration of the invention in any way. Rather, various changes may be made in the function and arrangement of the elements described in these embodiments without departing from the spirit and scope of the invention. Thus, the protected scope of the present invention is as set forth in the appended claims.

## Claims

1. A safety lancet device, comprising
a housing having a side hole and a front opening;
an actuating unit disposed on an outer surface of the housing;
a needle unit disposed inside the housing, the needle unit including:
a needle corresponding to the front opening;
a flex pin corresponding to the side hole, one end of the flex pin extending into the side hole to locate the needle unit at a first position; and
a blocking pin;
a blocking unit, disposed inside the housing, corresponding to the blocking pin to allow the needle unit to forwardly pass through the blocking unit and prevent the needle unit from backwardly passing through the blocking unit; and
an elastic unit connecting the needle unit and the housing;
wherein when an external force is exerted on the actuating unit, the actuating unit pushes the flex pin passing through the side hole and the elastic unit pushes the needle unit passing through the block unit to a second position.

2. The device according to claim 1, wherein the blocking unit has a guiding surface and a blocking surface, and wherein the guiding surface guides the blocking pin to allow the needle unit to forwardly move from the first position to the second position, and the blocking surface blocks the blocking pin to limit the needle unit backwardly moving from the second position to the first position.

3. The device according to claim 2, wherein the blocking unit protrudes from an inner surface of the housing to form a hook shape.

4. The device according to claim 1, wherein when the needle unit moves to the second position, the needle passes through the front opening and is exposed to outside environment of the housing.

5. The device according to claim 1, wherein one end of the actuating unit connects to the outer surface of the housing, and the other end of the actuating unit has a protrusion corresponding to the side hole for pushing the flex pin in response to the external force.

6. The device according to claim 1, further comprising a track disposed inside the housing, and the needle unit further comprising a bulge corresponding to the track to facilitate the movement of the needle unit.

7. The device according to claim 1, wherein the housing is assembled from a first housing and a second housing.

8. The device according to claim 1, wherein the elastic unit is a spring to provide an elastic force to push the needle unit.
